# EUROPÄISCHE PATENTSCHRIFT

(11) **EP 2 143 464 B1**
(45) Veröffentlichungstag und Bekanntmachung des Hinweises auf die Patenterteilung: **18.01.2017**
(21) Anmeldenummer: 09162536.8
(22) Anmeldetag: 12.06.2009
(51) Int. Cl.: A61N 1/05

(54) **Implantierbare Elektrodenleitung oder Elektrodenleitungsanordnung**
Implantable electrode lead or electrode lead device
Sonde électrode implantable ou appareil sonde électrode

(30) Priorität: 10.07.2008 DE 102008040304
(43) Veröffentlichungstag der Anmeldung: 13.01.2010
(60) Teilanmeldung: 16195063.9
(73) Patentinhaber: Biotronik CRM Patent AG, 6341 Baar (CH)
(72) Erfinder: Dörr, Thomas, 12437, Berlin (DE)
(74) Vertreter: Galander, Marcus

(56) Entgegenhaltungen:
- EP-A1- 1 516 644
- WO-A2-2006/105395
- WO-A2-2008/058265
- US-A- 5 728 140
- US-A1- 2008 082 132

## Beschreibung

Die Erfindung betrifft eine implantierbare Leitung oder Leitungsanordnung zur Implantation im linken Ventrikel eines Herzens unter Perforation des atrialen oder ventrikulären Septums.

Als Stand der Technik für eine linksventrikuläre Stimulation und Wahrnehmung ist derzeit die Implantation einer Elektrode in eine linksventrikuläre Vene via Koronarsinus anzusehen. Diese sog. Koronarsinuselektroden kommen vorwiegend für die kardiale Resynchronisationstherapie zum Einsatz.

In der medizinischen Literatur finden sich vermehrt Fallberichte von transseptaler Implantation linksventrikulären Stimulationselektroden zur Resynchronisationstherapie. Diese Implantationstechniken wurden immer unter Zuhilfenahme vorhandener Katheter, Führungsdrähte und Elektroden durchgeführt. Als Zugang zum linken Ventrikel wurden entweder die Punktion des atrialen Septums oder des Ventrikelseptums beschrieben, vgl. z.B. Transseptal endocardial left ventricular pacing: An alternative technique for coronary sinus lead placement in cardiac resynchronization therapy, van Gelder BM, Scheffer MG, Meijer A, et al, Heart Rhythm 2007 Apr; 4(4):454-60.

Ferner werden derzeit Konzepte zur transmuralen linksventrikulären Druckmessung in klinischen Studien untersucht. Hier werden Drucksensoren transmural im linken Ventrikel zur dauerhaften telemetrischen Druckmessung im linken Ventrikel platziert; vgl. unter www.transomamedical.com oder folgende Fachveröffentlichungen:
A Novel Technique For Assessing Load-Dependent Cardiac Function During LVAD Support Using Telemetered Left Ventricular Pressure. McConnell, PI, Del Rio, CL, Kwiatkowski, P, Farrar, D, Shipkowitz, T, Michler, RE, Sun, B. ASAIO Journal. 51(2): 31A, March/April 2005;
In Vivo Safety and Accuracy of a Clinically Applicable Telemetered Left Ventricular Pressure Module: Intermediate-Term Results. McConnell, PI, de Cunha, D, Shipkowitz, T, Van Hee, J, Long, P and Hamlin, R. Heart Failure Society Meeting, September 2004;
A System for Long-Term Measurement of Left Ventricular Pressure in Heart Failure Patients Living at Home. Sweitzer, N, Park, S. Heart Failure Society Meeting, September 2002:
   Automated Non-Invasive Monitoring of Left Ventricular Hemodynamics During Onset of Heart Failure in an Ambulatory Yucatan Mini Pig Model Using a New System Under Development for Assessing Heart Failure Patients at Home. Park, S, Sweitzer, N. Heart Failure Society Meeting, September 2002, oder
   A System For Long-Term Measurement Of Left Ventricular Pressure In Heart Failure Patients Living At Home. Park, S, Sweitzer, N and May, G. Heart Failure & Circulatory Support Summit, Cleveland, OH, August 2002.

Für angeborene Atriumsseptumdefekte, offene Foramen oder ovale und Ventrikelseptumdefekte werden derzeit eine Vielzahl von kommerziellen Verschlusssystemen (z.B. Premere™ PFO, SJM) angeboten, die mittels Kathetertechnik platziert werden können und einen zuverlässigen Verschluss des Septumdefektes gewährleisten; vgl. dazu Transcatheter patent foramen ovale closure using the premere PFO occlusion system. Andrea Donti, Alessandro Giardini, Luisa Salomone, Roberto Formigari, Fernando M. Picchio. Catheterization and Cardiovascular Interventions, vol 68/5 2006.

In WO 2006/105395 A2 wird eine transseptale/trans-myocardiale ventrikuläre Stimulationselektrode beschrieben.

Das Dokument US 2008/0082132 A1 offenbart eine transseptales Implantat.

In ca. 10-15% der Implantationen ist aufgrund der anatomischen Gegebenheiten die zuverlässige Implantation einer linksventrikulären Koronarsinus-Elektrode nicht möglich. Des Weiteren ist die Dislokationsrate von linksventrikulären Elektroden, die für die kardiale Resynchronisationstherapie (CRT) über den Koronarsinus implantiert würden, größer als die einer herkömmlichen rechtventrikulären Schrittmacherelektrode. Aus diesen Gründen ist derzeit die Einführung einer rein linksventrikulären Stimulation - unter Verwendung einer Koronarsinuselektrode - für die Bradykardietherapie oder die Implantation von automatischen Kardiovertern/Defibrillatoren (ICD) nicht gegeben, da sowohl der Implantationserfolg als auch die Sicherheit mit dieser Art linksventrikulärer Elektrode nicht gegeben ist. Ein weiterer Nachteil einer Koronarsinuselektrode sind die sehr eingeschränkten Platzierungsoptionen. Meist sind nur 1 bis 2 verschiedene Positionen zu Fixierung der Sonde gegeben. Dies wird als eine wesentliche Ursache für die z. T. schlechte Responderrate (60-70%) der CRT diskutiert.

Die weiter oben vorgestellten Techniken zur Elektrodenimplantation in den linken Ventrikel via atrialem oder ventrikulärem Septum sind sehr aufwändig und haben sich aufgrund der bestehen Risiken (RV Shunt, Thromben) bislang nicht durchgesetzt, obwohl hier eine freie Platzierung der Elektrode im linken Ventrikel möglich ist und damit die Nachteile der zuverlässigen Sondenfixierung, der Responderrate und anatomischen Einschränkungen behoben werden.

Die transmurale LV-Druckmessung stellt hier nur die Möglichkeit dar, durch den Herzmuskel ein System dauerhaft in den linken Ventrikel einzubringen. Allerdings wird hier eine sehr kurze Sonde mit einem Drucksensor in den linken Ventrikel gebracht, die nicht für die elektrische Stimulation des Herzens verwendet werden kann. Die in WO 2006/105395 A2 beschriebene Sonde ist jedoch derart konstruiert, dass aktive Stimulationsfläche nur im Bereich des links-ventrikulären Septums liegt und nicht im linken Ventrikel frei positionierbar ist. Weiter wird in WO 2006/105395 A2 nicht auf die Repositionierung und auf die Explantationsfähigkeit einer Elektrode eingegangen.

Um die oben genannten Nachteile zu beheben, besteht die Aufgabe der Erfindung darin, eine linksventrikuläre Sonde zu konstruieren, die durch das atriale oder ventrikuläre Septum vom rechten Ventrikel in den linken Ventrikel geschoben werden und frei innerhalb des linken Ventrikel manövriert und fixiert werden kann, wobei das Implantationsrisiko eines Links-Rechts-Shunts durch geeignete konstruktive Maßnahmen am Sondenkörper minimiert wird. Des Weiteren sind die Möglichkeit der Repositionierung einer solchen Sonde und deren Explantation zu berücksichtigen.

Diese Aufgabe wird durch eine implantierbare Leitungsanordnung gemäß Anspruch 1 gelöst. Zweckmäßige Fortbildungen des Erfindungsgedankens sind Gegenstand der abhängigen Ansprüche.

Die Erfindung schließt den wesentlichen Gedanken ein, an der Perforationsstelle im atrialen oder ventrikulären Septum, durch die die Elektrodenleitung verlegt wird, ein geeignetes Element vorzusehen, die zumindest während einer Einwachsphase eine zuverlässige gegenseitige Abdichtung der an das Septum angrenzenden Bereiche des Herzens sichert. In einer ersten Ausprägung des Erfindungsgedankens geschieht dies durch die Elektrodenleitung selbst, welche dann ein geeignetes Verschlusselement trägt. In einer relativ selbstständigen zweiten Ausprägung des Erfindungsgedankens ist hierzu ein separates Verschlusselement vorgesehen, welches vor der Einführung der Elektrodenleitung im Septum platziert und anschließend beim Verlegen der Elektrodenleitung von dieser durchstoßen wird.

Es wird ausdrücklich angemerkt, dass die Erfindung auch bei einer zum Einsatz im linken Atrium vorgesehenen Leitung bzw. Leitungsanordnung anwendbar ist, die durch ein Septum in das linke Atrium geführt ist.

Ein wichtiger Vorteil der erfindungsgemäßen Lösung ist der sichere und zuverlässige Zugang zum linken Ventrikel ohne die anatomischen Einschränkungen des Koronarsinus-Zugangs. Mit dieser Technik ist es dann möglich, primär linksventrikulär gesteuerte Herzschrittmacher, ICDs und CRT-Geräte anzubieten. Vorteil der primär linksgesteuerten Systeme sind:
- Physiologisch günstigerer Stimulationsort;
- bessere Sensingsignale aufgrund der größeren Muskelmasse;
- günstigere Bedingungen zu Sondenfixierung und geringeres Perforationsrisiko aufgrund der größeren Wandstärke;
- bessere Möglichkeiten der haemodynamischen Optimierung durch Stimulation;
- der Nachteil der RV-Stimulation wird weitgehend aufgehoben!
Gegenüber vorbekannten Lösungen ist zudem bei Verwendung der Durchführung ("Arbeitskanal") eine einfache Repositionierung und Explantation der transseptalen Sonde gegeben.

Der Verschlusskörper kann fest mit der Elektrode verbunden werden, so dass eine longitudinale Bewegung des Elektrodenkörpers im Septum vermieden wird.

Der Verschlusskörper ist in einer weiteren Ausführung ausgeprägt als expandierbares Schirmchen.

In einer ähnlichen Ausführung ist der Verschlusskörper ausgeprägt als ein expandierbares Schirmchen und als ein expandierbarer, auf der Elektrode verschiebbarer Anker (proximal des Schirmchens).

Die Sonde ist im Bereich des Verschlußkörpers bevorzugt beschichtet, um eine schnelle Bindegewebsbildung im Bereich der Perforationsstelle zu fördern.

Der "Arbeitskanal" hat bevorzugt einen maximalen freien Durchmesser von 2mm, wenn keine Elektrode durchgeschoben ist. (akzeptabler Links-Rechts Shunt).

Der "Arbeitskanal" ist in einer weiteren Ausführung mit Röntgenmarkern gekennzeichnet bzw. aus einem Röntgensichtbaren Material gefertigt.

Der "Arbeitskanal" wird z.B. durch expandierbare Fixatoren (wie z.B. Stents) im Septum befestigt. Auch die LV-Sonde kann im "Arbeitskanal" durch expandierbare Fixatoren befestigt werden. Diese Fixatoren sind an der Sonde angebracht. Die expandierbaren Fixatoren können zudem zur uni- und bipolaren Stimulation des Septums verwendet werden.

Vorteile und Zweckmäßigkeiten der Erfindung ergeben sich im Übrigen aus der nachfolgenden Beschreibung von Ausführungsbeispielen anhand der Figuren. Von diesen zeigen:
Fig. 1 die Gesamtansicht einer Defibrillationsanordnung mit einer Ausführungsform der Erfindung,
Fig. 2 eine Elektrodenleitung gemäß einer Ausführungsform der Erfindung,
Fig. 3 eine Elektrodenleitung gemäß einer alternativen Ausführungsform der Erfindung,
Fig. 4A bis 4C Darstellungen einer weiteren Ausführung der Erfindung, in verschiedenen Realisierungsphasen, und
Fig. 5 eine Detailansicht einer weiteren Ausführungsform der Erfindung.

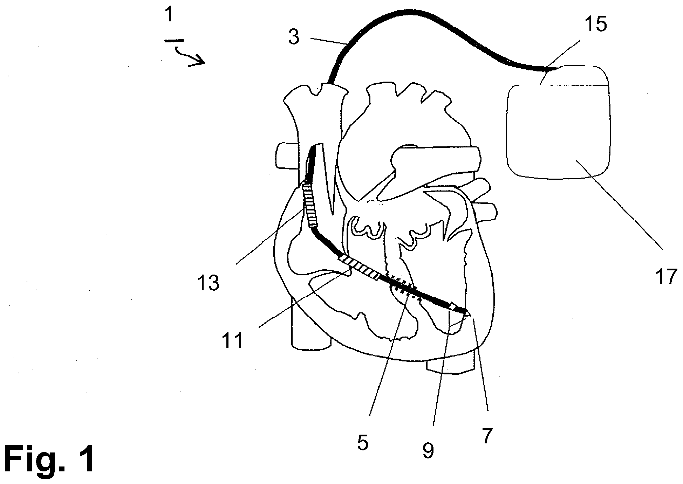

In Fig. 1 ist eine erfindungsgemäße Anordnung 1 dargestellt. Eine linksventrikuläre Elektrodenleitung 3 ist dabei durch eine implantierbare transseptale Durchführung ["Arbeitskanal"] 5 in den linken Ventrikel vorgeschoben und dort mittels einer konventionellen Schraube 7 fixiert. In der gezeigten Ausführung hat die linksventrikuläre Elektrodenleitung 3 eine bipolare Stimulationselektrode 9 und zusätzlich zwei Schockelektroden (distal: 11, proximal 13). Der Durchmesser der distalen Schockelektrode 11 ist so gewählt, dass diese nicht durch die Durchführung 5 in den linken Ventrikel rutschen kann. Die Leitung 3 ist mit einem Elektrodenstecker 15 (IS-4 bzw. IS-1 und DF-1) mit einem implantierbaren Defibrillator 17 verbunden.

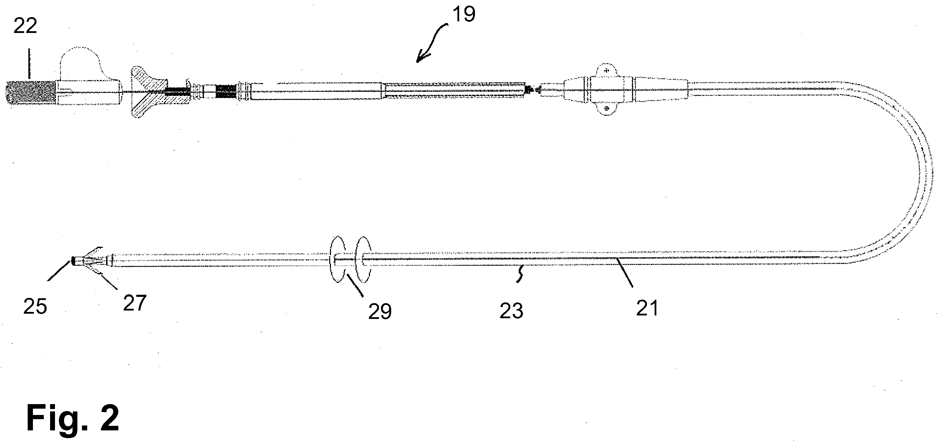

Fig. 2 zeigt als weitere Ausführungsform der Erfindung speziell eine Elektrodenleitung 19, die über einen Führungsdraht 21 mit proximaler Handhabe 22 implantiert werden kann und deren Aufbau mit einem flexiblen (hier U-förmig gebogen dargestellten) Elektrodenleitungskörper 23, einer Tip-Elektrode 25 und distalen Fixierungs-Widerhaken 27 zur Verankerung in der Wandung des Ventrikels an sich bekannt ist. Neu an dieser Elektrodenleitung ist ein - hier symbolisch als zwei Dichtscheiben mit kleinem Abstand voneinander dargestelltes - Verschlusselement 29 zum Verschluss einer Perforationsstelle im Septum, über die die Elektrodenleitung (wie in Fig. 1 gezeigt) in den linken Ventrikel geführt ist.

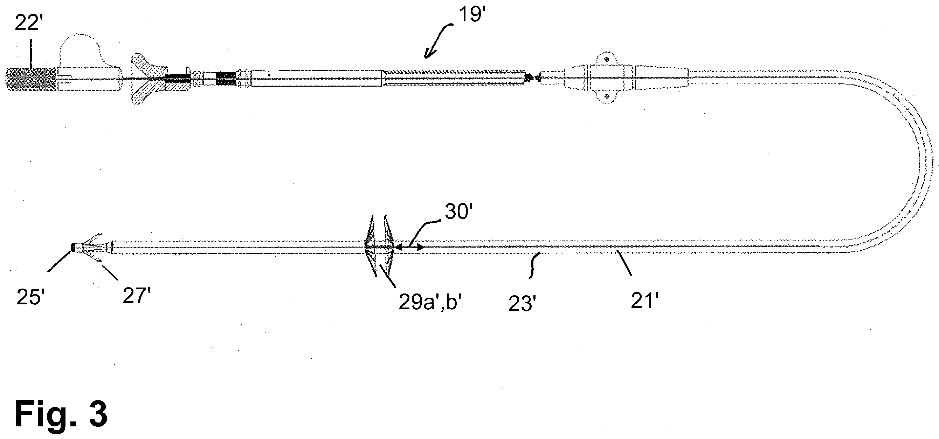

Fig. 3 zeigt als spezielle Ausgestaltung dieser Ausführung eine Elektrodenleitung 19', bei der das Verschlusselement 29 durch zwei gegensinnig aufspreizbare Schirme 29a' 29b' gebildet ist, die dank eines zusätzlichen Steuerdrahtes 30' neben dem Führungsdraht 21, nach Einführung der Elektrodenleitung beidseitig der Perforationsstelle im Septum aufgespreizt werden können und im aufgespreizten Zustand die Umgebung des Durchstoßpunktes abdichten.

Das proximale Schirmchen kann mittels des zusätzlichen Mandrins 30' zusätzlich auf der Sonde verschoben werden, so dass das Ventrikelseptum zwischen den beiden Schirmchen fixiert wird. Die beiden Schirmchen 29a', 29b' können dabei auch als (z.B. bipolare) aktive Stimulationselektroden eingesetzt werden.

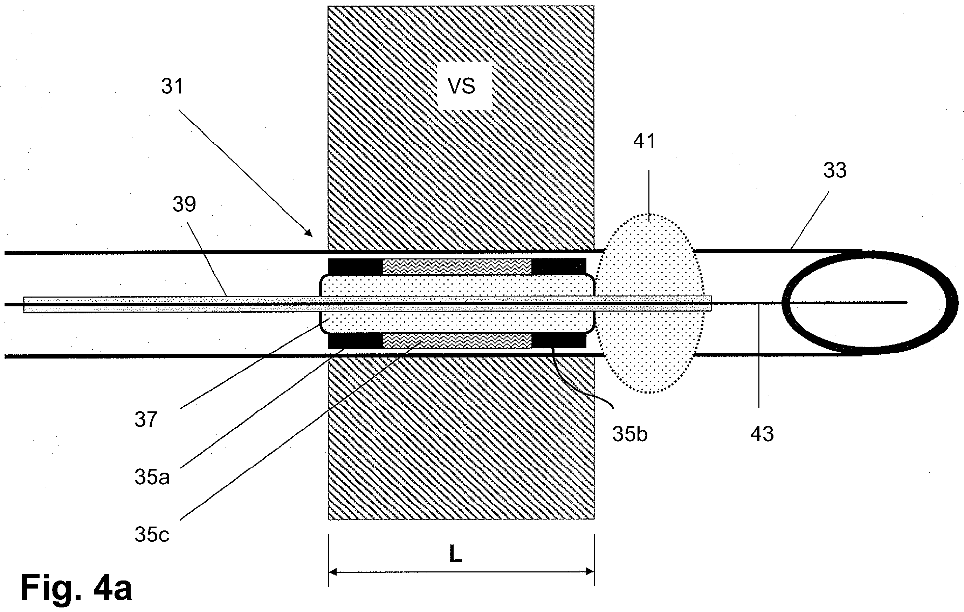

Die in Fig. 4A-C dargestellte septale Durchführung ("Arbeitskanal") 31 wird zunächst mittels einer Brockenbrough-Nadel 33 durch Punktion des Ventrikelseptums VS hergestellt. Die Durchführung selbst besteht aus 2 expandierbaren Stents 35a, 35b, eingebettet in einen flexiblen biokompatiblen Schlauch 35c, z.B. Silikon oder Polyurethan. Diese Durchführung ist auf einem expandierbaren Ballon 37 eines Ballonkatheters 39 montiert und wird durch Ballonexpansion im Septum fixiert.

Um die korrekte Position der Durchführung zu gewähleisten, befindet sich auf dem Ballonkatheder ein zweiter Ballon 41 größeren Durchmessers, der nach Punktion des linken Ventrikels und Zurückziehen der Brockenbrough-Nadel expandiert wird. Anschließend wird der Ballonkatheter so weit zurückgezogen, dass der größere Ballon 41 am Ventrikelseptum VS anliegt. Danach wird die Durchführung 31, deren Länge L vorab mittels Echokardiographie bestimmt wurde, durch die Expansion des kleineren Ballons 37 fixiert, indem der expandierende Ballon 37 die Stens 35a, 35b mit dem Schlauchabschnitt 35c dazwischen gegen die Wandung des durch die Brockenbrough-Nadel gebildeten Loches im Septum rückt.

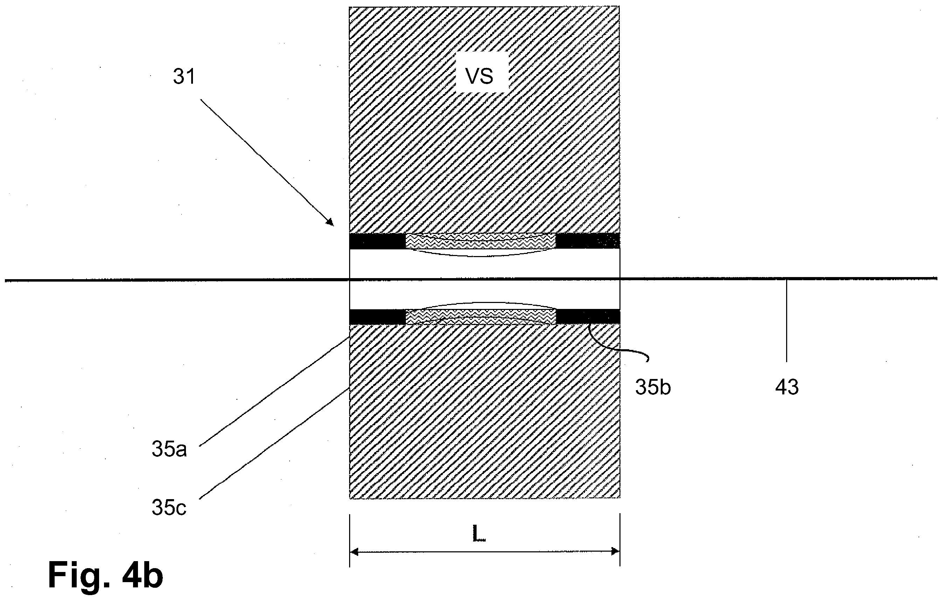

Nach dem Entfernen des Ballonkatheters 39 verbleibt die Durchführung 31 und ggf. ein Führungsdraht 43 in der Punktionsstelle (Fig 4B). Die beiden Stents 35a, 35b sind dabei im Röntgenbild sichtbar, so dass eine anschließende Navigation der LV-Sonde durch diese transseptale Durchführung einfach möglich ist. Das Links-Rechts-Shuntvolumen wird dabei durch den flexiblen Teil der Durchführung 31 minimiert. Wenn keine Leitung eingeführt ist, wird nämlich die lichte Weite d durch ein Sich-Nach-Innen-Wölben des flexiblen Schlauchabschnitts 35c zwischen den Stens 35a, 35b reduziert.

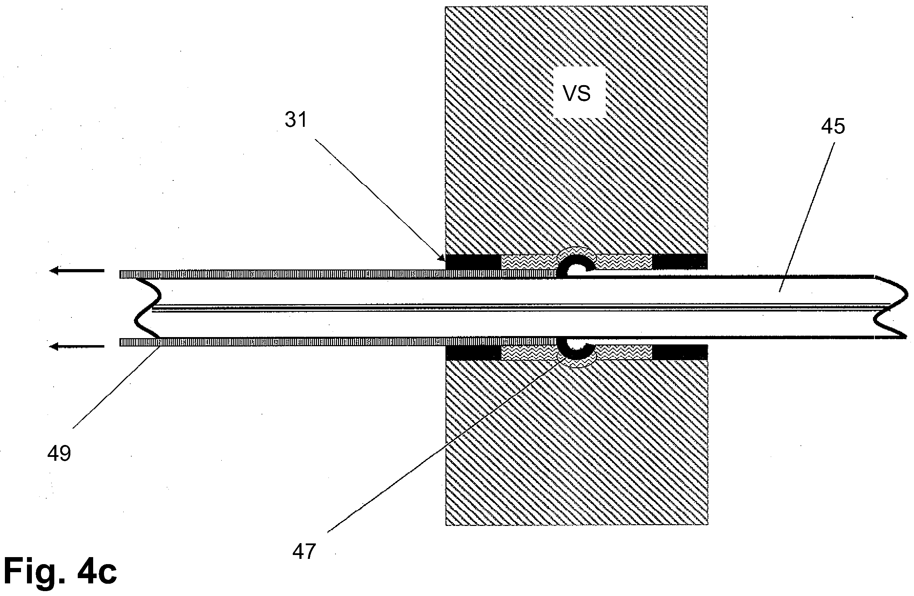

In der Fig. 4C ist die oben beschriebene transseptale Durchführung 31 zusammen mit einer linksventrikulären Leitung bzw. Sonde 45 dargestellt. Um eine Längsbewegung der Sonde 45 innerhalb der transseptalen Durchführung zu vermeiden, kann sie durch einen selbstexpandierenden Fixator 47 innerhalb der Durchführung befestigt werden. Dieser Fixator ist in dem Ausführungsbeispiel als ein röntgensichtbares Federelement realisiert, das durch Zurückziehen eines Schlauches 49 freigegeben wird und so die Elektrodenleitung in der Durchführung festklemmt. Auf diese Weise wird ein Abrieb der Elektrodenleitung oder des Fixators vermieden.

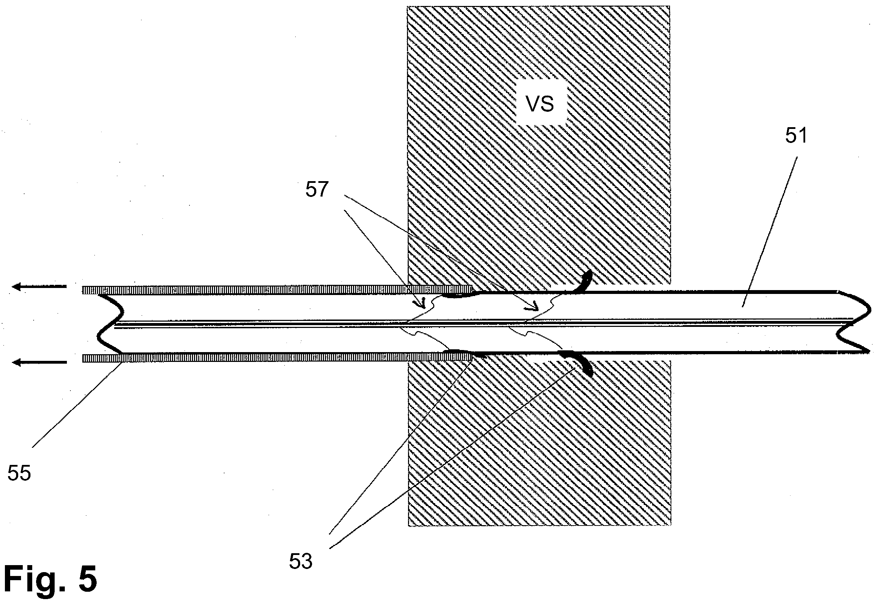

Alternativ zu den weiter oben beschriebenen LV-Sonden in Kombination mit einer Durchführung ist in Fig. 5 eine LV-Sonde 51 dargestellt, die selbstständig, ohne separates Verschlusselement mittels direkt angebrachter Fixatoren 53 im Septum befestigt werden kann, wobei diese Fixatoren 53 zunächst durch einen Schlauch 55 ummantelt sind und durch das Zurückziehen dieses Schlauches dann durch Federn 57 im Septum VS expandiert werden.

Der Schlauch 55 ist dabei derart ausgeführt, dass er nach der Implantation (z.B. durch Peeling) vollständig von der Elektrodenleitung entfernt werden kann. Für den Fall einer Explantation einer solchen Elektrodenleitung können die Fixatoren durch einen entsprechenden Schlauch - nach Abschneiden des Elektrodensteckers - wieder "eingefahren" werden. Die Fixatoren können als aktive Stimulationselektroden eingesetzt werden, indem sie mit den elektrischen Zuleitungen 57 der Elektrodenleitung verbunden sind. Die Implantation in dieser Leitung erfolgt ebenfalls mittels Brockenbrough-Nadel.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Implantierbare Leitungsanordnung zur Implantation im linken Ventrikel eines Herzens unter Perforation des atrialen oder ventrikulären Septums, mit einer Leitung (45), die einen langgestreckten, flexiblen Leitungskörper und eine Elektrode (9, 25) und/oder einen Sensor und/oder eine Medikamentenapplikationseinrichtung am oder nahe einem distalen Ende des Leitungskörpers aufweist, und mit
einem separaten Verschlusselement (31) zur Abdichtung der Perforationsstelle, welches zur Fixierung im Septum ausgebildet ist und ein an den Außendurchmesser der Leitung angepasstes Lumen aufweist,
**dadurch gekennzeichnet, dass**
das Verschlusselement eine implantierbare septale Durchführung bestehend aus zwei ballonexpandierbaren Stents (35a, 35b), eingebettet in einen flexiblen biokompatiblen Schlauch (35c), umfasst.

2. Leitungsanordnung nach Anspruch 1, **dadurch gekennzeichnet, dass** der Schlauch (35c) aus Silikon oder Polyurethan besteht.

3. Leitungsanordnung nach Anspruch 1, **dadurch gekennzeichnet, dass** die Stents (35a, 35b) im Röntgenbild sichtbar sind.

4. Leitungsanordnung nach einem der vorhergehenden Ansprüche, **dadurch gekennzeichnet, dass** die lichte Weite des Lumens ohne eingeführte Leitung maximal 2 mm beträgt.

5. Leitungsanordnung nach Anspruch 1, **dadurch gekennzeichnet, dass** die Leitung (45) mehrere Abfühl- und/oder Stimulationselektroden aufweist.

6. Leitungsanordnung nach Anspruch 1 oder 5, **dadurch gekennzeichnet, dass** mindestens ein Abschnitt des Verschlusselementes und/oder ein Abschnitt des Leitungskörpers nahe dem Verschlusselement mit einem eine schnelle Bindegewebsbildung im Bereich der Perforationsstelle fördernden Wirkstoff in einer Form beschichtet ist, die den Wirkstoff während einer Einwachsphase wirksam werden lässt.

7. Leitungsanordnung nach einem der Ansprüche 1, 5 oder 6, **dadurch gekennzeichnet, dass** der Leitungskörper Mittel zur Fixierung (47, 53) der Leitung im separaten Verschlusselement aufweist.

8. Leitungsanordnung nach Anspruch 7, **dadurch gekennzeichnet, dass** die Mittel zur Fixierung (47, 53) selbstexpandierend sind.

9. Leitungsanordnung nach Anspruch 7 oder 8, **dadurch gekennzeichnet, dass** die Mittel zur Fixierung (47, 53) röntgensichtbare Federelemente sind, die durch Zurückziehen oder Entfernen eines Schlauches (49, 55) freigegeben werden.

10. Leitungsanordnung nach einem der Ansprüche 7 bis 9, **dadurch gekennzeichnet, dass** die Mittel zur Fixierung (47, 53) zugleich als Stimulationselektrode zur elektrischen Stimulation des Septums ausgebildet sind.

11. Leitungsanordnung nach einem der vorhergehenden Ansprüche, mit einer Leitung, welche am oder nahe dem distalen Ende des Leitungskörpers angeordnete Fixierungsmittel (7, 27, 27') zur Fixierung der Leitung in oder an einer Wand des linken Ventrikels und/oder mehrere Abfühl- und/oder Stimulationselektroden aufweist.

## Claims

1. An implantable line arrangement for implantation in the left ventricle of a heart with perforation of the atrial or ventricular septum, said line arrangement comprising a line (45), which comprises an elongate, flexible line body and an electrode (9, 25) and/or a sensor and/or a drug application device at or near a distal end of the line body,
and comprising
a separate closure element (31) for sealing the perforation site, which closure element is designed to be fixed in the septum and has a lumen adapted to the outer diameter of the line,
**characterised in that**
the closure element comprises an implantable septal feedthrough consisting of two balloon-expandable stents (35a, 35b), embedded in a flexible biocompatible tube (35c).

2. The line arrangement according to claim 1, **characterised in that** the tube (35c) consists of silicone or polyurethane.

3. The line arrangement according to claim 1, **characterised in that** the stents (35a, 35b) are visible in an X-ray image.

4. The line arrangement according to any one of the preceding claims, **characterised in that** the clear width of the lumen without inserted line is at most 2 mm.

5. The line arrangement according to claim 1, **characterised in that** the line (45) has a plurality of sensing and/or stimulation electrodes.

6. The line arrangement according to claim 1 or 5, **characterised in that** at least a portion of the closure element and/or a portion of the line body near to the closure element are/is coated with an active substance which promotes rapid development of connective tissue in the region of the perforation site, said coating being provided in a form which allows the active substance to be effective during an ingrowth phase.

7. The line arrangement according to any one of claims 1, 5 or 6, **characterised in that** the line body comprises means for fixing (47, 53) the line in the separate closure element.

8. The line arrangement according to claim 7, **characterised in that** the fixing means (47, 53) are self-expanding.

9. The line arrangement according to claim 7 or 8, **characterised in that** the fixing means (47, 53) are X-ray-visible spring elements which are released by retracting or removing a tube (49, 55).

10. The line arrangement according to any one of claims 7 to 9, **characterised in that** the fixing means (47, 53) are formed simultaneously as stimulation electrodes for electrical stimulation of the septum.

11. The line arrangement according to any one of the preceding claims, having a line, which comprises fixing elements (7, 27, 27') arranged at or near the distal end of the line body in order to fix the line in or on a wall of the left ventricle and/or a plurality of sensing and/or stimulation electrodes.

## Revendications

1. Système de ligne implantable pour l'implantation dans le ventricule gauche d'un coeur sous perforation du septum atrial ou ventriculaire, avec une ligne (45) qui présente un corps de ligne allongé, souple et une électrode (9, 25), et/ou un capteur, et/ou un dispositif d'application de médicament sur ou à proximité de l'extrémité distale du corps de ligne,
et avec
un élément de fermeture (31) séparé pour l'étanchéité du point de perforation, lequel est conçu pour la fixation sur le septum et présente une lumière ajustée au diamètre extérieur de la ligne,
**caractérisée en ce que**
l'élément de fermeture comprend un passage septal implantable constitué de deux stents (35a, 35b) pouvant s'expanser en ballonnets, incorporés dans un tuyau (35c) biocompatible souple.

2. Système de ligne selon la revendication 1, **caractérisée en ce que** le tuyau (35c) est constitué de silicone ou de polyuréthane.

3. Système de ligne selon la revendication 1, **caractérisée en ce que** les stents (35a, 35b) sont visibles par l'image en radiographie X.

4. Système de ligne selon l'une des revendications précédentes, **caractérisée en ce que** la largeur intérieure de la lumière se situe au maximum à 2 mm sans ligne insérée.

5. Système de ligne selon la revendication 1, **caractérisée en ce que** la ligne (45) présente plusieurs électrodes de détection et/ou de stimulation.

6. Système de ligne selon la revendication 1, ou 5, **caractérisée en ce qu'**au moins une section de l'élément de fermeture, et/ou une section du corps de ligne proche de l'élément de fermeture, est revêtue avec une matière active favorisant une formation rapide de tissu conjonctif dans la zone du point de perforation, qui fait que la matière active devient efficace pendant une phase de croissance interne.

7. Système de ligne selon l'une des revendications 1, 5 ou 6, **caractérisée en ce que** le corps de ligne présente des moyens pour la fixation (47, 53) de la ligne dans l'élément de fermeture séparé.

8. Système de ligne selon la revendication 7, **caractérisée en ce que** les moyens pour la fixation (47, 53) sont auto-expansibles.

9. Système de ligne selon la revendication 7, ou 8, **caractérisée en ce que** les moyens pour la fixation (47, 53) sont des éléments de ressort visibles aux rayons X qui peuvent être libérés par le retrait ou l'enlèvement d'un tuyau (49, 55).

10. Système de ligne selon l'une des revendications 7 à 9, **caractérisée en ce que** les moyens pour la fixation (47, 53) sont conçus simultanément sous la forme d'une électrode de stimulation pour la stimulation électrique du septum.

11. Système de ligne selon l'une des revendications précédentes, avec une ligne, laquelle présente des éléments de fixation (7, 27, 27') disposés sur ou à proximité de l'extrémité distale du corps de ligne pour la fixation de la ligne dans ou sur une paroi du ventricule gauche, et/ou plusieurs électrodes de détection et/ou de stimulation.
